# EUROPEAN PATENT SPECIFICATION

(11) **EP 3 225 238 B1**
(45) Date of publication and mention of the grant of the patent: **29.12.2021**
(21) Application number: 15862163.1
(22) Date of filing: 27.11.2015
(51) Int. Cl.: A61K 31/352, A61K 31/353, A61K 8/49, A61P 17/00, A61Q 19/00

(54) **COMPOSITION FOR INDUCING FACILITATION OF CELL REJUVENATION COMPRISING GENISTEIN OR EPIGALLOCATECHIN GALLATE**
ZUSAMMENSETZUNG ZUR INDUZIERUNG VON ZELLVERJÜNGUNG MIT GENISTEIN ODER EPIGALLOCATECHINGALLAT
COMPOSITION POUR INDUIRE LA FACILITATION DE RAJEUNISSEMENT CELLULAIRE COMPRENANT DE LA GÉNISTÉINE OU ÉPIGALLO-CATÉCHINE GALLATE

(30) Priority: 28.11.2014 KR 20140168532
(43) Date of publication of application: 04.10.2017
(73) Proprietor: Amorepacific Corporation, Seoul 140-777 (KR)
(72) Inventor: LEE, Eun Kyung, Yongin-si Gyeonggi-do 17074 (KR); GOH, Myeong Jin, Yongin-si Gyeonggi-do 17074 (KR); CHOI, Hyun Jung, Yongin-si Gyeonggi-do 17074 (KR); CHO, Eun Gyung, Yongin-si Gyeonggi-do 17074 (KR); LEE, Tae Ryong, Yongin-si Gyeonggi-do 17074 (KR)
(74) Representative: Bugnion Genève
(86) International application number: PCT/KR2015/012881
(87) International publication number: WO 2016/085302

(56) References cited:
- WO-A1-2004/082697
- WO-A2-2010/118419
- KR-A- 20130 057 634
- US-A1- 2014 186 315
- ZHU J ET AL: "Intervention of EGCG on cell senescence and chronic photodamage in human skin fibroblasts", EMBASE,, 1 January 2008 (2008-01-01), XP002564623,
- DONG-WOOK HAN ET AL: "Preventive Effects of Epigallocatechin-3- O -Gallate against Replicative Senescence Associated with p53 Acetylation in Human Dermal Fibroblasts", OXIDATIVE MEDICINE AND CELLULAR LONGEVITY, vol. 2012, 1 January 2012 (2012-01-01), pages 1-13, XP055481956, US ISSN: 1942-0900, DOI: 10.1155/2012/850684
- FRANCESCA POLITO ET AL: "Genistein aglycone, a soy-derived isoflavone, improves skin changes induced by ovariectomy in rats : Genistein improves skin changes in OVX rats", BRITISH JOURNAL OF PHARMACOLOGY, vol. 165, no. 4, 25 January 2012 (2012-01-25), pages 994-1005, XP055481965, UK ISSN: 0007-1188, DOI: 10.1111/j.1476-5381.2011.01619.x
- KIM, D. ET AL.: 'Epigallocatechin-3-Gallate and Hinokitiol Reduce Melanin Synthesisvia Decreased MITF Production' ARCHIVES OF PHARMACAL RESEARCH vol. 27, no. 3, 2004, pages 334 - 339, XP055445673
- KHITKA, B. ET AL.: 'Antioxidant Properties of Puerarin and Genistein from White Kwao Krua Induced by Elicitors and Their Antihyperglycemic Effect on Rats' SUMNAREE. J. SCI. TECHNOL. vol. 17, no. 1, 2009, pages 27 - 37, XP055445677
- LEE, SOO - YEON ET AL.: 'Down-regulation of Tyrosinase, MITF, TRP-1, and TRP-2 Expressions by Juniperus rigida Sieb. in Murine B16F10 Metanoma' JOURNAL OF LIFE SCIENCE vol. 23, no. 12, 2013, pages 1445 - 1453, XP053031163
- TROMPEZINSKI, S. ET AL.: 'Comparative Effects of Polyphenols from Green Tea (EGCG) and Soybean (Genistein) on VEGF and IL -8 Release from Normal Human Keratinocytes Stimulated with the Proinflammatory Cytokine TNF a' ARCHIVES OF DERMATOLOGICAL RESEARCH vol. 295, 2003, pages 112 - 116, XP055445696
- None

## Description

### [Technical Field]

The present disclosure discloses a novel non-therapeutic cosmetic or food use of genistein and epigallocatechin gallate (EGCG).

### [Background Art]

To prevent degenerative diseases and live long healthily by delaying senescence is what all people want. Efforts to find substances that delay senescence have been made from the past to the present. The causes that accelerate senescence include oxidation by reactive oxygen species such as superoxides, hydrogen peroxide, hydroxyl radical and singlet oxygen.

Free radicals produced from metabolism of the reactive oxygen species cause oxidative damage in vivo by acting on proteins, biological membranes, DNAs, etc. To prevent this, a lot of researches are actively made inside and outside Korea on existing or new antioxidants.

However, because senescence is affected not only by oxidation but also by various factors including telomeres, cellular replicative activity, genetic damage, recovering ability, and etc., a method of using the cell senescence is necessary to evaluate the ability of preventing cellular senescence of natural products and medicines, not just to evaluate their antioxidant effects.

Cellular senescence refers the phenomenon by which normal somatic cells cease to divide after a certain number of divisions. It is an important mechanism which contributes to aging of an individual and tissues, to inhibit abnormal proliferation of cells and carcinogenesis. Cellular senescence is caused by telomere shortening, which is located in the distal end of a chromosome, after repeated division of somatic cells, increased activity of oncogenes or antioncogenes, excessive oxidative stress, UV or radioactive radiation, cytotoxic substances such as anticancer drugs, inflammatory responses, etc.

Cellular senescence not only contributes to aging of an individual and tissues, but also plays an important role in the onset of various diseases. Aged cells are frequently observed in inflammatory tissues in rheumatoid arthritis, osteoarthritis, hepatitis, chronic skin damage, arteriosclerosis, etc. Cellular senescence is also observed in prostatic hyperplasia, hepatitis, liver cancer, etc.

When the aged cells are accumulated, the damaged tissue cannot be recovered properly because the aged cells do not divide well. In addition, they accelerate the tissue damage by secreting enzymes that degrade nearby tissues, inflammatory cytokines, etc., thereby contributing to the onset of aging-associated diseases.

Because cellular senescence is regarded as an essential cause of aging, there have been efforts to develop a method of delaying cellular senescence. For example, there have been efforts to explore substances that can regulate cellular senescence and utilize them to prevent and treat diseases.

The representative substances which have been found to regulate cellular senescence include resveratrol which is abundant in red wine and is known to increase the activity of SIRT1, retinoic acid which delays cellular senescence in keratinocytes, etc. Also, there have been efforts to identify and isolate substances capable of regulating cellular senescence from plant extracts to utilize them in cosmetics, health functional foods, etc.

However, the previous studies focus only on the method of preventing or delaying senescence and a method of actively rejuvenating aged cells to young and healthy cells has never been reported.

(Non-patent document 1) Harman, D., Free radical theory of aging: Role of free radicals in the origination and evolution of life, aging and disease processes, 3-49. Alan R Liss, New York (1986)

WO 2010/118419 A2 discloses agents that can maintain the cell cycle-arrested state in senescent and post mitotic cells. This document refers to epigallocatechin gallate (EGCG) from Green Tea Extract (GTE) and Grape Seed Extract (GSE).

### [Disclosure]

### [Technical Problem]

In an aspect, the present disclosure is directed to providing a use of genistein, epigallocatechin gallate (EGCG), derivatives thereof, isomers thereof, pharmaceutically acceptable salts thereof, prodrugs thereof, hydrates thereof and solvates thereof for inducing rejuvenation of cells.

In another aspect, the present disclosure is directed to reducing the expression of factors related to increase of melanin producing ability.

In another aspect, the present disclosure is directed to inducing rejuvenation of cells by regulating the expression of enzymes involved in melanin production.

### [Technical Solution]

The present invention provides a non-therapeutic cosmetic or food use of a compound comprising one or more selected from a group consisting of genistein, epigallocatechin gallate (EGCG), pharmaceutically acceptable salts thereof, hydrates thereof and solvates thereof, for inducing rejuvenation of melanocytes.

### [Advantageous Effects]

According to the present invention, a non-therapeutic cosmetic or food use of a compound comprising one or more selected from a group consisting of genistein, epigallocatechin gallate (EGCG), pharmaceutically acceptable salts thereof, hydrates thereof and solvates thereof reduces the expression of factors related to increase of melanin producing ability including tyrosinase, reduces the expression of senescence markers including p16 and p21, and regulates the expression of enzymes involved in melanin production including histone deacetylase 5 and EZH1 (enhancer of zeste homolog 1), and thus may have an effect of rejuvenating melanocytes.

### [Brief Description of Drawings]

FIGs. 1A-1D show a result of measuring the change in the expression of factors related to increase of melanin producing ability after subculturing melanocytes in a medium containing genistein or epigallocatechin gallate (EGCG).
FIG. 2 shows a result of observing the change in senescence markers (p16 and p21) after subculturing melanocytes in a medium containing genistein or epigallocatechin gallate (EGCG).
FIG. 3 shows a result of observing the change in the expression of enzymes involved in melanin production after subculturing melanocytes in a medium containing genistein or epigallocatechin gallate (EGCG).
FIGs. 4A-4B show a result of observing the change the phenotype of melanocytes after subculturing melanocytes in a medium containing genistein or epigallocatechin gallate (EGCG). FIG. 4A shows a result of microscopic observation and FIG. 4B shows the proportion of aged cells to total melanocytes.

In each figure, PA means each passage, PA11E means melanocytes of passage 11 which have been cultured in a medium containing EGCG from passage 7, PA11G means melanocytes of passage 11 which have been cultured in a medium containing genistein from passage 7, PA12E means melanocytes of passage 12 which have been cultured in a medium containing EGCG from passage 7 and PA12G means melanocytes of passage 12 which have been cultured in a medium containing genistein from passage 7.

### [Best Mode for Carrying Out Invention]

Hereinafter, the present disclosure is described in detail.

In an aspect, the present disclosure provides a composition for inducing rejuvenation of cells, which comprises one or more selected from a group consisting of genistein, epigallocatechin gallate (EGCG), derivatives thereof, isomers thereof, pharmaceutically acceptable salts thereof, prodrugs thereof, hydrates thereof and solvates thereof.

In the present disclosure, a "derivative" may mean a compound which is obtained by substituting a part of a specific compound with another atom or group of atoms. In the present disclosure, an "isomer" includes, in particular, not only optical isomers (e.g., essentially pure enantiomers, essentially pure diastereomers or mixtures thereof), but also conformational isomers (i.e., isomers different only in the angles of one or more chemical bonds), position isomers (especially, tautomers) or geometric isomers (e.g., cis-trans isomers).

In the present disclosure, "essentially pure" means, for example, when used in connection with enantiomers or diastereomers, that a particular compound as an example of the enantiomer or diastereomer is present in amount of about 90% (w/w) or more, specifically about 95% or more, more specifically about 97% or more or about 98% or more, further more specifically about 99% or more, even more specifically about 99.5% or more.

In the present disclosure, "pharmaceutically acceptable" means that it can be approved or is approved by a regulatory agency of the government or an international organization, or that it is listed in the Pharmacopoeia or is recognized as other general pharmacopoeia for use in animals, more specifically in human, since significant toxic effect can be avoided when used with a common medicinal dosage.

In the present disclosure, a "pharmaceutically acceptable salt" means a salt of an aspect of the present disclosure, which is pharmaceutically acceptable and exhibits the desired pharmacological activity of its parent compound. The salt may include (1) an acid addition salt formed from an inorganic acid such as hydrochloric acid, hydrobromic acid, sulfuric acid, nitric acid, phosphoric acid, etc. or an organic acid such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2,2,2]-oct-2-ene-1-carboxylic acid, glucoheptonic acid, 3-phenylpropionic acid, trimethylacetic acid, tert-butylacetic acid, lauryl sulfuric acid, gluconic acid, glutamic acid, hydroxynaphthoic acid, salicylic acid, stearic acid and muconic acid; or (2) a salt formed when an acidic proton in the parent compound is substituted.

In the present disclosure, a "prodrug" means a drug which has been chemically changed to regulate its physical and chemical properties, it does not exhibit physiological activity as it is, but exerts medicinal effect after it is converted to the original drug through chemical or enzymatic action in vivo.

In the present disclosure, a "hydrate" means a compound to which water is bonded. The term is used in a broad sense, including an inclusion compound which lacks chemical bonding with water.

In the present disclosure, a "solvate" means a higher-order compound formed between a molecule or ion of solute, and a molecule or ion of solvent.

In the present disclosure, genistein may mean one of the isoflavones which is a phytochemical compound contained in plants. It may be present in soybean, pagoda tree, clover or other plant species. Genistein may mean a substance having a structure of Chemical Formula 1 below.

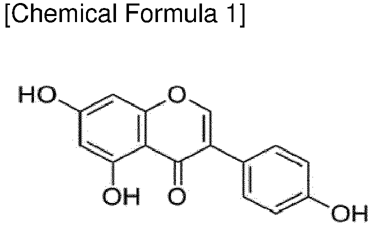

In the present disclosure, a genistein derivative may be a compound of formula below, but is not limited to, selected from a group consisting of each of R₁ to R₄ is independently selected from hydrogen, hydroxy, alkyl or substituted alkyl, phenyl or substituted phenyl, diphenylethyl or substituted diphenylethyl, thiophenyl or substituted thiophenyl, alkoxy, cycloalkyl, cycloalkoxy, heterocycloalkyl, aryloxy, haloalkoxy, aryl, haloalkyl, methoxy, benzyl or substituted benzyl, benzoyl, oxo, alkyidenyl, carbonyl, acyl, alkenyl, alkylamino and dialkylamino. For example, the genistein derivative of the present disclosure may be, but is not limited to, 6"-O-acetylgenistin, 6"-O-malonylgenistin, 6-carboxymethylgenistein or sophoricoside.

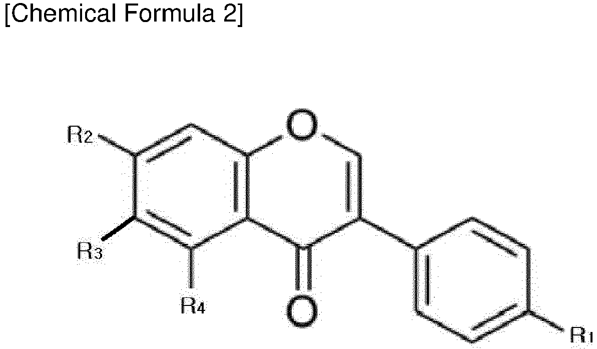

In the present disclosure, epigallocatechin gallate may mean a substance having a structure of Chemical Formula 3.

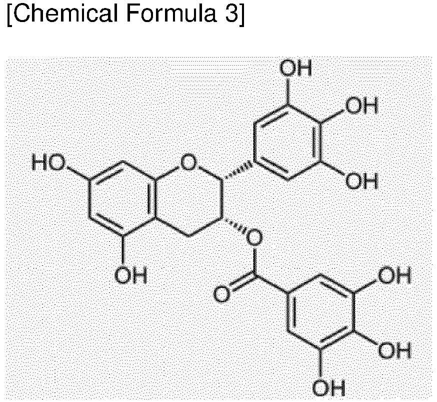

Furthermore, the derivative of epigallocatechin gallate of the present disclosure may be, but is not limited to, selected from a group consisting of (-)-epicatechin gallate (ECG), gallic acid (GA), (-)-epigallocatechin (EGC), (-)-epicatechin (EC) and gallocatechin (GC).

In an aspect of the present disclosure, the cell lines may be melanocytes.

In the present disclosure, "rejuvenation of cells" may mean restoring aged cells to young and healthy cells.

In the present disclosure, "melanocyte cell line" may mean a line of cloned melanocytes that can divide or proliferate continuously through culturing, carrying on the line.

In the present disclosure a "subculture system" may mean a system comprising one or more of a melanocyte mother cell line and a cell line obtained by subculturing a specific cell line for one to several passages.

In the present disclosure, a "melanocyte mother cell line" means a melanocyte cell line prior to subculturing.

In the present disclosure "early" and "late" are classified based on the number of subculturing of a cell line, For example, an "early cell line " may mean a PA1 cell line which is a melanocyte mother cell line and at least one of PA2, PA3, PA4, PA5 and PA6 cell line which have been obtained by subculturing the melanocyte mother cell line 1, 2, 3, 4, and 5 times, respectively, and a "late cell line " may mean at least one of PA7, PA8, PA9, PA10, PA11 and PA12 cell line which have been obtained by subculturing the melanocyte mother cell line 6, 7, 8, 9, 10 and 11 times, respectively. The early cell line may include a first early melanocyte cell line and a second early melanocyte cell line, and the late melanocyte cell line may include a first late melanocyte cell line and a second late melanocyte cell line. The first early melanocyte cell line may include at least one of PA1, PA2 and PA3 cell lines and the second early melanocyte cell line may include at least one of PA4, PA5 and PA6 melanocyte cell lines. Furthermore, the first late melanocyte cell line may include at least one of PA7, PA8 and PA9 cell lines and the second late melanocyte cell line may include at least one of PA10, PA11 and PA12 melanocyte cell lines.

In the present disclosure, passage n or PAn means a cell line which has been obtained by subculturing the mother cell line n - 1 times. For example, passage 3 or PA3 means a cell line which has been obtained by subculturing the mother cell line 2 times. As used herein, the n is an integer.

In this aspect, the rejuvenation of cells may mean restoring cells of a late cell line to those identical or similar to that of an early cell line. For example, it may mean restoration of a PA12 cell line to a PA1 cell line, a PA12 cell line to a PA2 cell line, a PA12 cell line to a PA3 cell line, a PA12 cell line to a PA4 cell line, a PA12 cell line to a PA5 cell line, a PA12 cell line to a PA6 cell line, a PA11 cell line to a PA1 cell line, a PA11 cell line to a PA2 cell line, a PA11 cell line to a PA3 cell line, a PA11 cell line to a PA4 cell line, a PA11 cell line to a PA5 cell line, a PA11 cell line to a PA6 cell line, a PA10 cell line to a PA1 cell line, a PA10 cell line to a PA2 cell line, a PA10 cell line to a PA3 cell line, a PA10 cell line to a PA4 cell line, a PA10 cell line to a PA5 cell line, a PA10 cell line to a PA6 cell line, a PA9 cell line to a PA1 cell line, a PA9 cell line to a PA2 cell line, a PA9 cell line to a PA3 cell line, a PA9 cell line to a PA4 cell line, a PA9 cell line to a PA5 cell line or a PA9 cell line to a PA6 cell line.

In a composition according to an aspect of the present disclosure, the composition may restore cells corresponding to cell lines which has been subcultured 6 or more times, to cells corresponding to cell lines which has been subcultured 5 or less times, of cell lines included in a subculture system. the subculture system may comprise, but is not limited to, an early melanocyte cell line comprising one or more selected consisting of a PA1, PA2, PA3, PA4, PA5 and PA6 cell lines, wherein the PA1 is a melanocyte mother cell line, and the PA2, PA3, PA4, PA5 and PA6 have been obtained by subculturing the mother cell line 1, 2, 3, 4 and 5 times, respectively; and a late melanocyte cell line including at least one selected consisting of PA7, PA8, PA9, PA10, PA11 and PA12 cell line which has been obtained by subculturing the melanocyte mother cell line 6, 7, 8, 9, 10 and 11 times, respectively;

In a composition according to an aspect of the present disclosure, the subculture system may be consisted of, but not limited to, PA1, PA2, PA3, PA4, PA5, PA6, PA7, PA8, PA9, PA10, PA11 and PA12 cell lines.

In this aspect, the subcultured cell line may be a cell line with an accession number KCTC 12906BP or KCTC 12907BP. For example, the early melanocyte cell line may be a cell line with the accession number KCTC 12906BP and the late melanocyte cell line may be a cell line with the accession number KCTC 12907BP. Specifically, the PA3 cell line may be a cell line with the accession number KCTC 12906BP and the PA11 cell line may be a cell line with the accession number KCTC 12907BP.

In a composition according to an aspect of the present disclosure, the composition comprising one or more selected from a group consisting of genistein, epigallocatechin gallate (EGCG), derivatives thereof, isomers thereof, pharmaceutically acceptable salts thereof, prodrugs thereof, hydrates thereof and solvates thereof, may reduce, but is not limited to, the expression of one or more selected from a group consisting of tyrosinase, microphthalmia-associated transcription factor (MITF), tyrosinase-related protein-1 (TRP-1), tyrosinase-related protein-2 (TRP-2), EZH1 (enhancer of zeste homolog 1), p16 and p21. In this aspect, although the expression level of the above-described substances including tyrosinase may increase as the subculturing is repeated, a cell line that has been subcultured in a medium containing the composition according to an aspect of the present disclosure (e.g., from passage 7) may exhibit the expression level of one or more selected from a group consisting of tyrosinase, microphthalmia-associated transcription factor (MITF), tyrosinase-related protein-1, tyrosinase-related protein-2, EZH1, p16 and p21, which is comparable to or lower than that of an early cell line(e.g., from passage 2).

In an aspect of the present disclosure, the composition may reduce the expression of one or more selected from a group consisting of p14, p19, p27, cyclin D, cyclin E, cyclin-dependent kinase 4 (CDK4), cathelin-related antimicrobial peptide (CRAMP), stathmin, EF-1a and chitinase 3-like 3. In this aspect, although the expression level of the above-described substances including p14 may increase as the subculturing is repeated, a cell line that has been subcultured in a medium containing the composition according to an aspect of the present disclosure (e.g., from passage 7) may exhibit the expression level of p14, etc, which is comparable to or lower than that of an early cell line.

In a composition according to an aspect of the present disclosure, the composition may increase the expression of histone deacetylase 5.

The detection of the change in expression may be made, but not limited to, in tissues, cells (e.g., melanocytes) or body fluids (e.g., blood or tissue fluid).

In a composition according to an aspect of the present disclosure, the composition may be a pharmaceutical, health food or cosmetic composition.

Specifically, the composition may be administered to a patient in an appropriate unit dosage form according to a method common in the pharmaceutical or cosmetic field. Dosage forms suitable for this purpose include an injection, a formulation for topical application, etc. as parenteral formulations. Specifically, the formulation for injection may be an isotonic aqueous solution or a suspension. However, a commonly used diluent or excipient such as a filler, an extender, a binder, a wetting agent, a disintegrant, a surfactant, etc., may also be included. The composition of the present disclosure may be administered parenterally, for example, directly into a specific site, according to a common method. The injection of the composition may be made using a syringe. It should be understood that the actual administration dosage of the active ingredient is determined in consideration of various related factors including the administration route, body weight, age and sex of the patient, and the like. The concentration of the composition included in a single unit dosage may be, for example, 1 µM. However, the scope of the present disclosure is not limited by the administration dosage by any means.

In a composition according to an aspect of the present disclosure, the composition may be a composition for topical application.

In a composition according to an aspect of the present disclosure, the composition may be a cosmetic composition. Specifically, the cosmetic composition may be in the form of a base cosmetic, a makeup cosmetic, a hair cosmetic, a body cosmetic, etc., whose formulation is not particularly limited and may be selected appropriately depending on purposes.

For example, the cosmetic composition may be formulated into, but not limited to, a solution, a suspension, an emulsion, a paste, a gel, a cream, a lotion, a powder, a soap, a surfactant-containing cleanser, an oil, a powder foundation, an emulsion foundation, a wax foundation, a spray, etc. More specifically, it may be formulated into a base cosmetic such as a softening lotion, a nourishing lotion, a lotion, a body lotion, a nourishing cream, a massage cream, a moisturizing cream, a hand cream, an essence, an eye cream, a cleansing cream, a cleansing foam, a cleansing water, a pack, a gel, a patch, an oil-in-water (O/W) or water-in-oil (W/O) emulsion, etc., a coloring cosmetic such as a lipstick, a makeup base, a foundation, etc., a cleanser such as a shampoo, a rinse, a body cleanser, a toothpaste, a mouthwash, etc., or a hair cosmetic such as a hair tonic, a hair gel, a hair mousse, a hair restorer, a hair dye, etc..

The cosmetic composition contains a cosmetically acceptable medium or base. It can be provided in the form of any formulation suitable for topical application, for example, a solution, a gel, a solid, an anhydrous paste, an oil-in-water emulsion, a suspension, a microemulsion, a microcapsule, a microgranule, or an ionic (liposome) and/or nonionic vesicular dispersion, or in the form of a cream, a skin lotion, a lotion, a powder, an ointment, a spray or a conceal stick. These formulations can be prepared by a method commonly employed in the art.

When the formulation of the present disclosure is a solution or an emulsion, a solvent, a solubilizer or an emulsifier is used as a carrier ingredient. For example, water, ethanol, isopropanol, ethyl carbonate, ethyl acetate, benzyl alcohol, benzyl benzoate, propylene glycol, 1,3-butyl glycol oil, a glycerol aliphatic ester, polyethylene glycol or a fatty acid ester of sorbitan is used.

When the formulation of the present disclosure is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, or tragacanth, etc. may be used as a carrier ingredient.

When the formulation of the present disclosure is a paste, a cream or a gel, an animal oil, a vegetable oil, a wax, paraffin, starch, tragacanth, a cellulose derivative, polyethylene glycol, silicone, bentonite, silica, talc, zinc oxide, etc., may be used as a carrier ingredient.

When the formulation of the present disclosure is a powder or a spray, lactose, talc, silica, aluminum hydroxide, calcium silicate, polyamide powder, etc. may be used as a carrier ingredient. In particular, when the formulation is a spray, it may further contain a propellant such as chlorofluorohydrocarbon, propane/butane or dimethyl ether.

In an exemplary embodiment of the present disclosure, the cosmetic composition may further contain a thickener. The thickener included in the cosmetic composition of the present disclosure may be methyl cellulose, carboxymethyl cellulose, carboxymethyl hydroxyguanine, hydroxymethyl cellulose, hydroxyethyl cellulose, carboxyvinyl polymer, polyquaternium, cetearyl alcohol, stearic acid, carrageenan, etc. Preferably, one or more of carboxymethyl cellulose, carboxyvinyl polymer and polyquaternium may be used. Most preferably, carboxyvinyl polymer may be used.

In an exemplary embodiment of the present disclosure, the cosmetic composition may contain various matrices and additives if necessary. The kinds and amounts of these ingredients may be readily selected by those of ordinary skill. If necessary, the cosmetic composition may further include acceptable adjuvants, for example, an antiseptic, a pigment, an additive, etc., commonly used in the art.

Specifically, the antiseptic may be phenoxyethanol, 1,2-hexanediol, or etc., and a fragrance may be a synthetic fragrance.

And, in an exemplary embodiment of the present disclosure, the cosmetic composition may comprise one or more selected from a group consisting of a watersoluble vitamin, an oil-soluble vitamin, a polypeptide, a polysaccharide, a sphingolipid and a seaweed extract. In addition, an oil and fat, a humectant, an emollient, a surfactant, an organic or inorganic pigment, an organic powder, a UV absorbent, an antiseptic, a sterilizer, an antioxidant, a plant extract, a pH control agent, an alcohol, a pigment, a fragrance, a blood circulation promoter, a cooling agent, an antiperspirant, purified water, etc., may be further added as a compound.

The health food may mean, but not limited to a food which is prepared by using nutrients that may be insufficient in daily diets or ingredients useful to the human body (functional ingredients), and which maintains and improves health by maintaining the normal function of human body or activating physiological functions. The health food may be prepared and processed, but not limited to, in the form of a tablet, a capsule, a powder, a granule, a liquid, a pill, etc. The health food can be prepared and processed in the form according to the law.

In an aspect of the present disclosure, the health drink composition may further contain other ingredients in addition to the essential active ingredient. The health drink composition has no specific limitation of containing other ingredients, except containing the compounds above, as essential ingredients, in directed ratio. The health drink composition may contain various flavors, natural carbohydrates, etc. as in common drinks. The natural carbohydrate may be a monosaccharide, a polysaccharide, a sugar such as cyclodextrin, etc., or a sugar alcohol such as xylitol, sorbitol, erythritol, etc. As the flavor other than the flavor described above, a natural flavor (thaumatin, stevia extract (e.g., rebaudioside A, glycyrrhizin, etc.)) and a synthetic flavor (e.g., saccharin, aspartame, etc.) may be used.

In general, the amount of the active ingredient administered via the health food composition may be from 0.0001 mg/kg/day to about 1000 mg/kg/day, more specifically from 0.02 mg/kg/day to 100 mg/kg/day. The administration may be made once or several times a day.

The additionally contained ingredients are not limited to those described above and any ingredient may be added within a range of not negatively affecting the purpose and effect of the present disclosure.

According to the present invention, it is provided a non-therapeutic cosmetic or food use of a compound comprising one or more selected from a group consisting of genistein, epigallocatechin gallate (EGCG), pharmaceutically acceptable salts thereof, hydrates thereof and solvates thereof, for inducing rejuvenation of melanocytes.

According to an aspect of the present invention, the compound of the non-therapeutic cosmetic or food use of the invention may comprise 0.001-2.0 µM, preferably 0.005-1.5 µM of genistein, epigallocatechin gallate (EGCG), pharmaceutically acceptable salts thereof, hydrates thereof or solvates thereof. Specifically, in an aspect of the present disclosure, the concentrations of the genistein, epigallocatechin gallate, pharmaceutically acceptable salts thereof, hydrates thereof or solvates thereof, comprised in the compound, may be, but not limited to, at least 0.0001 µM, at least 0.001 µM, at least 0.002 µM, at least 0.003 µM, at least 0.004 µM, at least 0.005 µM, at least 0.006 µM, at least 0.007 µM, at least 0.008 µM, at least 0.009 µM, at least 0.01 µM, at least 0.15 µM, at least 0.15 µM, at least 0.2 µM, at least 0.25 µM, at least 0.3 µM, at least 0.35 µM, at least 0.4 µM, at least 0.45 µM, at least 0.5 µM, at least 0.6 µM, at least 0.7 µM, at least 0.8 µM, at least 0.9 µM, at least 1.0 µM, at least 1.1 µM, at least 1.2 µM, at least 1.3 µM, at least 1.4 µM, at least 1.5 µM, at least 1.6 µM, at least 1.7 µM, at least 1.8 µM, at least 1.9 µM, or at least 2.0 µM, and may be, but not limited to, up to 2.1 µM, up to 2.0 µM, up to 1.9 µM, up to 1.8 µM, up to 1.7 µM, up to 1.6 µM, up to 1.5 µM, up to 1.4 µM, up to 1.3 µM, up to 1.2 µM, up to 1.1 µM, up to 1.0 µM, up to 0.9 µM, up to 0.85 µM, up to 0.80 µM, up to 0.75 µM, up to 0.70 µM, up to 0.65 µM, up to 0.60 µM, up to 0.55 µM, up to 0.50 µM, up to 0.45 µM, up to 0.40 µM, up to 0.35 µM, up to 0.30 µM, up to 0.25 µM, up to 0.20 µM, up to 0.15 µM, up to 0.10 µM, up to 0.05 µM, up to 0.04 µM, up to 0.03 µM, up to 0.02 µM, up to 0.01 µM, up to 0.005 µM, up to 0.004 µM, up to 0.003 µM, up to 0.002 µM, up to 0.001 µM, up to 0.0005 µM.

Hereinafter, the present disclosure will be described in detail through examples. However, the following examples are for illustrative purposes only and the scope of the present disclosure is not limited by the examples.

### [Example 1] Subculturing of melanocytes

Human primary melanocytes (Life Technologies, CA, USA) were cultured in a M-254 medium (Gibco BRL, NY, USA) supplemented with a human melanocyte growth supplement (HMGS; Gibco BRL, NY, USA) in a 5% CO₂ incubator at room temperature on a 100-mm culture plate while replacing the medium every other day until the cells occupy 80% of the plate area and then subcultured on another 100-mm culture plate at a ratio of 1:4.

The melanocytes first placed on the 100-mm culture plate were considered as passage 1 (PA1) and subculturing was performed 11 times (PA12).

### [Example 2] Observation of change in expression of factors related to increase of melanin producing ability by melanocytes

The effect of genistein and epigallocatechin gallate (EGCG) on the late melanocyte cell line was investigated based on the change in the expression level of factors known to be related to melanin production (tyrosinase, microphthalmia-associated transcription factor (MITF), tyrosinase-related protein-1 (TRP-1) and tyrosinase-related protein-2 (TRP-2)).

As a control group, melanocytes were subcultured to passage 11 and passage 12 without any treatment, and as a test group, the cells were subcultured under the same environment as the control group until passage 6, and then subcultured from passage 7 to passage 11 and passage 12 in a medium containing 1 µM genistein or epigallocatechin gallate (EGCG).

Specifically, Q-PCR was conducted in order to investigate the relationship of the change in the expression level of the factors known to be related to melanin production (tyrosinase, microphthalmia-associated transcription factor (MITF), tyrosinase-related protein-1 (TRP-1) and tyrosinase-related protein-2 (TRP-2)). cDNAs were synthesized from RNAs isolated from the human melanocytes, and the change in the quantity of mRNAs were measured by Q-PCR (Applied Biosystems, 7500 Fast) using respective primers. The Q-PCR experiment was conducted by repeating 40 cycles of 15 seconds at 95 ºC and 60 seconds at 60 ºC. The increased gene expression relative to that of the control group was measured. The following TaqMan primers manufactured by Applied Biosystems were used: tyrosinase (TYR, Product No.: Hs01099965_m1), MITF (Product No.: Hs01117294_m1), TRP1 (Product No.: Hs00167051_m1), TRP2 (Product No.: Hs01095856_m1).

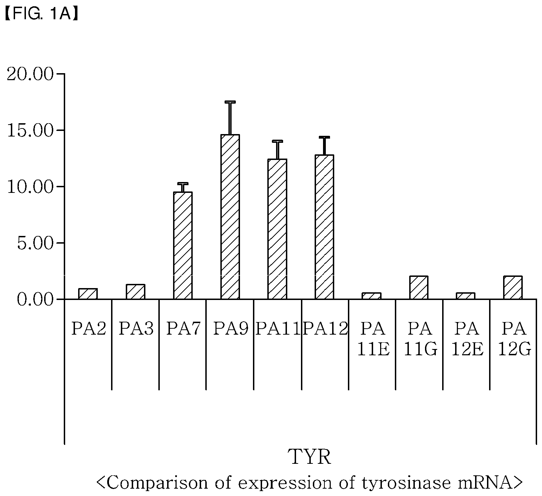

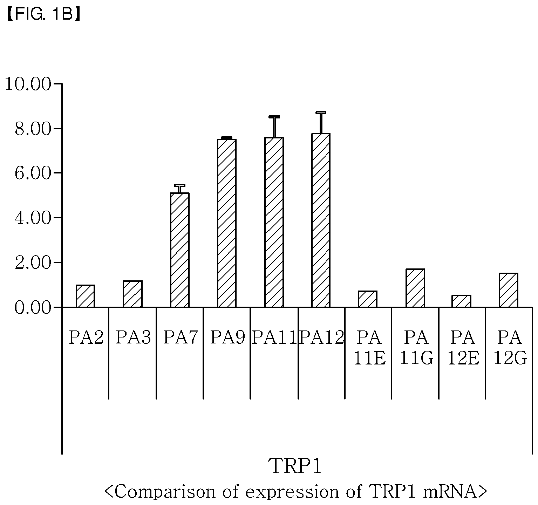

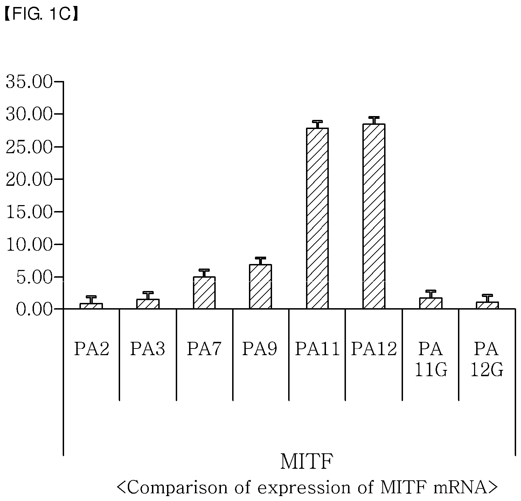

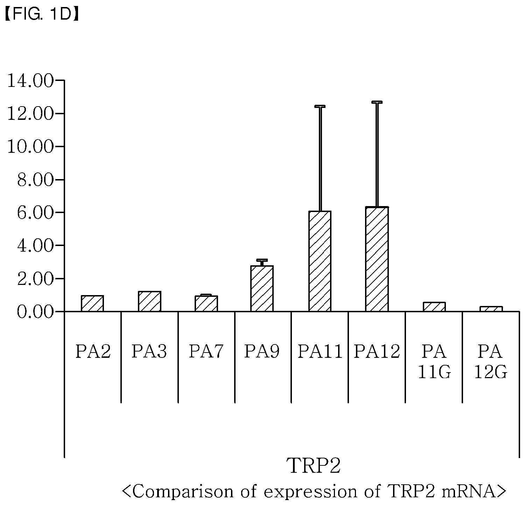

As a result (FIGs. 1A-1D), it was investigated that mRNA expression level of tyrosinase, microphthalmia-associated transcription factor (MITF), tyrosinase-related protein-1 (TRP-1) and tyrosinase-related protein-2 (TRP-2) of the late cell line of the test group was decreased significantly, while that of the control group was increased(in the graphs, the ordinate is the gene expression level/GAPDH mRNA expression level for each gene as a relative expression level of the corresponding gene).

### [Example 3] Observation of senescence markers of melanocytes

It was investigated whether the expression level of p16 and p21 which are found at high levels in aged cells is affected by genistein or epigallocatechin gallate (EGCG).

A control group and a test group were the same as in Example 2.

Specifically, cDNAs were synthesized from RNAs of melanocytes of different passages, and the change in the amount of mRNAs was measured by Q-PCR (Applied Biosystems, 7500 Fast) using primers specific for p16 and p21. The following TaqMan primers manufactured by Applied Biosystems were used: p16 (Product No.: Hs00923894_m1), p21 (Product No.: Hs00355782_m1).

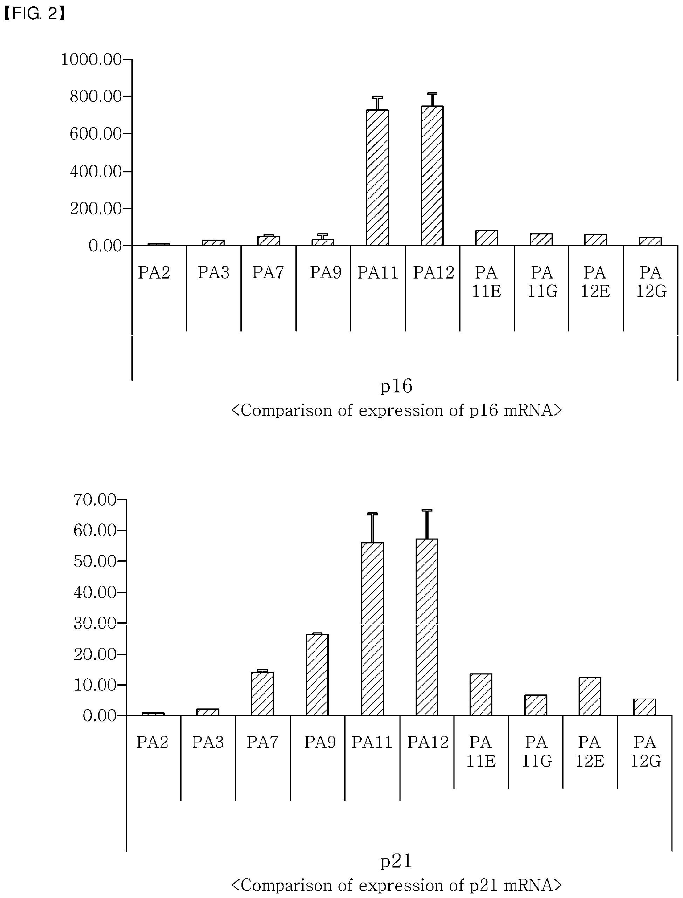

As a result (FIG. 2), the expression of p16 and p21 in the late cell line was greatly increased in the control group, but was significantly decreased in the test group (in the graphs, the ordinate is the gene expression level/GAPDH mRNA expression level for each gene as a relative expression level of the corresponding gene).

### [Example 4] Observation of change in expression of enzymes involved in melanin production

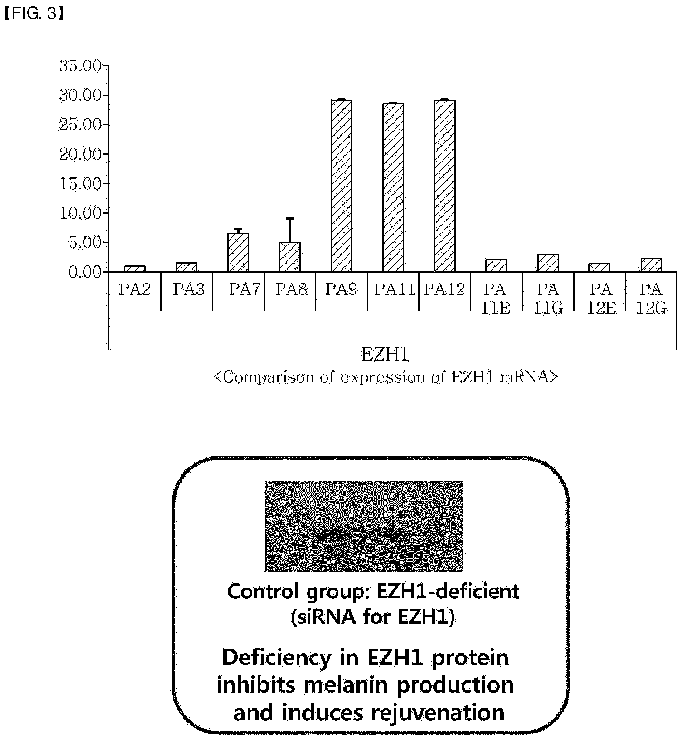

In order to investigate the effect of the EZH1 (enhancer of zeste homolog 1) protein on melanin production, the expression of the EZH1 protein was inhibited using siRNAs. As a result, melanin production was greatly decreased, which suggests that EZH1 inhibits melanin production (FIG. 3, bottom).

It was also investigated whether the expression level of EZH1 is affected by genistein or epigallocatechin gallate (EGCG).

A control group and a test group were the same as in Example 2.

Specifically, cDNAs were synthesized from RNAs of melanocytes of the control group and the test group, and the change in the amount of mRNAs was measured by Q-PCR (Applied Biosystems, 7500 Fast) using a primer specific for EZH1. The following TaqMan EZH1 primer manufactured by Applied Biosystems was used: EZH1 (Product No.: Hs00940463_m1).

As a result (FIG. 3), the expression of EZH1 by the late cell line was greatly increased in the control group, but was significantly decreased in the test group (in the graphs, the ordinate is the gene expression level/GAPDH mRNA expression level for each gene as a relative expression level of the corresponding gene).

### [Example 5] Observation of phenotype of melanocytes

In order to investigate whether genistein or epigallocatechin gallate (EGCG) affects the phenotype of melanocytes, the phenotype of the melanocytes of the control group and the test group was observed with an optical microscope (magnification spec. : 400X). The phenotype of cells included in the medium was observed with an optical microscope at 40X magnification.

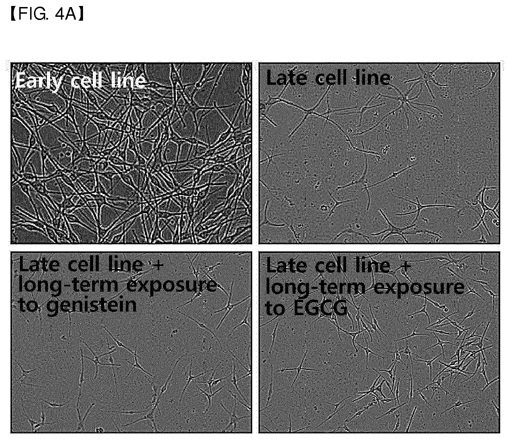

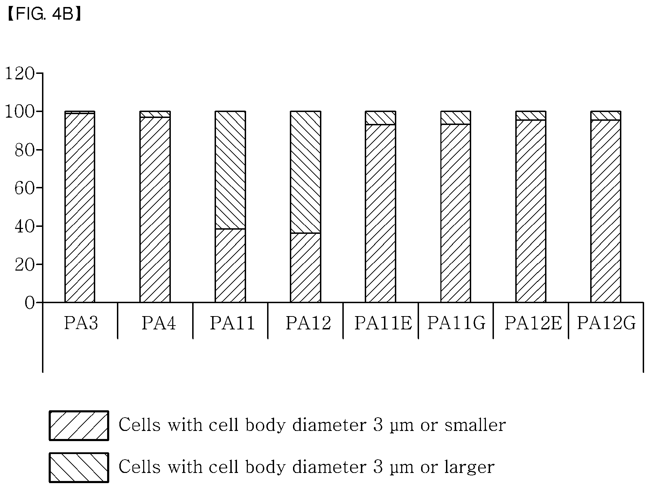

As can be seen in FIGs. 4A-4B, It was also investigated that the phenotype of the melanocytes of the late cell line became flat, increased in cell body size, and increased in the number of the dendrites and heterogeneity(number of cells with changed shape due to aging increased; number of cells with cell body diameter of at least 3 µm increased), however, after long-term exposure to genistein or epigallocatechin gallate (EGCG), the cells were recovered similarly to the cells of the early stage of subculturing.

Hereinafter, the present disclosure will be described in detail through formulation examples. However, the following formulation examples are for illustrative purposes only and the scope of the present disclosure is not limited by the formulation examples.

### [Formulation Example 1] Ointment

**[Table 1]**

| Ingredients | Contents (wt%) |
|---|---|
| Genistein or epigallocatechin gallate | 0.1 |
| Glycerin | 8.0 |
| Butylene glycol | 4.0 |
| Liquid paraffin | 15.0 |
| β-Glucan | 7.0 |
| Carbo mer | 0.1 |
| Caprylic/capric triglyceride | 3.0 |
| Squalane | 1.0 |
| Cetearyl glucoside | 1.5 |
| Sorbitan stearate | 0.4 |
| Cetearyl alcohol | 1.0 |
| Beeswax | 4.0 |
| Antiseptic, pigment and fragrance | Adequate |
| Purified water | Balance |

### [Formulation Example 2] Massage cream

**[Table 2]**

| Ingredients | Contents (wt%) |
|---|---|
| Genistein or epigallocatechin gallate | 1.5 |
| Glycerin | 8.0 |
| Butylene glycol | 4.0 |
| Liquid paraffin | 45.0 |
| β-Glucan | 7.0 |
| Carbo mer | 0.1 |
| Caprylic/capric triglyceride | 3.0 |
| Beeswax | 4.0 |
| Cetearyl glucoside | 1.5 |
| Sorbitan sesquioleate | 0.9 |
| Vaseline | 3.0 |
| Paraffin | 1.5 |
| Antiseptic, pigment and fragrance | Adequate |
| Purified water | Balance |

### [Formulation Example 3] Softening lotion (skin lotion)

**[Table 3]**

| Ingredients | Contents (wt%) |
|---|---|
| Genistein or epigallocatechin gallate | 0.1 |
| Glycerin | 3.0 |
| Butylene glycol | 2. |
| Propylene glycol | 2.0 |
| Carboxyvinyl polymer | 0.1 |
| PEG-12 nonyl phenyl ether | 0.2 |
| Polysorbate 80 | 0.4 |
| Ethanol | 10.0 |
| Triethanolamine | 0.1 |
| Antiseptic, pigment and fragrance | Adequate |
| Purified water | Balance |

### [Formulation Example 4] Nourishing lotion (milk lotion)

**[Table 4]**

| Ingredients | Contents (wt%) |
|---|---|
| Genistein or epigallocatechin gallate | 0.5 |
| Glycerin | 3.0 |
| Butylene glycol | 3.0 |
| Propylene glycol | 3.0 |
| Carboxyvinyl polymer | 0.1 |
| Beeswax | 4.0 |
| Polysorbate 60 | 1.5 |
| Caprylic/capric triglyceride | 5.0 |
| Squalane | 5.0 |
| Sorbitan sesquioleate | 1.5 |
| Liquid paraffin | 0.5 |
| Cetearyl alcohol | 1.0 |
| Triethanolamine | 0.2 |
| Antiseptic, pigment and fragrance | Adequate |
| Purified water | Balance |

### [Formulation Example 5] Hair pack

**[Table 5]**

| Ingredients | Contents (wt%) |
|---|---|
| Genistein or epigallocatechin gallate | 0.1 |
| Glycerin | 4.0 |
| Polyvinyl alcohol | 15.0 |
| Hyaluronic acid extract | 5.0 |
| β-Glucan | 7.0 |
| Allantoin | 0.1 |
| Nonyl phenyl ether | 0.4 |
| Polysorbate 60 | 1.2 |
| Ethanol | 6.0 |
| Antiseptic, pigment and fragrance | Adequate |
| Purified water | Balance |

### [Formulation Example 6] Injection

**[Table 6]**

| Ingredients | Contents (ppm) |
|---|---|
| Genistein or epigallocatechin gallate | 10 |
| Sterile distilled water for injection | Adequate |
| pH control agent | Adequate |

While the exemplary embodiments have been shown and described, it will be understood by those skilled in the art that various changes in form and details may be made thereto without departing from the spirit and scope of this disclosure as defined by the appended claims.

### [Accession Numbers]

Depository authority: Korea Research Institute of Bioscience and Biotechnology
Accession number: KCTC 12906BP
Date of accession: Sep. 17, 2015
Depository authority: Korea Research Institute of Bioscience and Biotechnology
Accession number: KCTC 12907BP
Date of accession: Sep. 17, 2015

## Claims

1. A non-therapeutic cosmetic or food use of a compound comprising one or more selected from a group consisting of genistein, epigallocatechin gallate (EGCG), pharmaceutically acceptable salts thereof, hydrates thereof and solvates thereof, for inducing rejuvenation of melanocytes.

2. The non-therapeutic use according to claim 1, wherein the compound comprises 0.001 to 2.0µM, preferably 0.005 to 1.5µM of genistein, epigallocatechin gallate (EGCG), pharmaceutically acceptable salts thereof, hydrates thereof or solvates thereof.

## Patentansprüche

1. Nichttherapeutische kosmetische oder Nahrungsmittelverwendung einer Verbindung, die eines oder mehrere umfasst, die aus einer Gruppe ausgewählt sind, die aus Genistein, Epigallocatechingallat (EGCG), pharmazeutisch verträglichen Salzen davon, Hydraten davon und Solvaten davon besteht, zum Herbeiführen einer Verjüngung von Melanozyten.

2. Nichttherapeutische Verwendung nach Anspruch 1, wobei die Verbindung 0,001 bis 2,0 µM, vorzugsweise 0,005 bis 1,5 µM, Genistein, Epigallocatechingallat (EGCG), pharmazeutisch verträgliche Salze davon, Hydrate davon oder Solvate davon umfasst.

## Revendications

1. Utilisation alimentaire ou cosmétique non thérapeutique d'un composé comprenant un ou plusieurs choisis dans le groupe constitué par la génistéine, le gallate d'épigallocatéchine (EGCG), leurs sels pharmaceutiquement acceptables, leurs hydrates et leurs solvates, pour induire un rajeunissement des mélanocytes.

2. Utilisation non thérapeutique selon la revendication 1, dans laquelle le composé comprend 0,001 à 2,0 µM, de préférence 0,005 à 1,5 µM de génistéine, de gallate d'épigallocatéchine (EGCG), de leurs sels pharmaceutiquement acceptables, de leurs hydrates ou de leurs solvates.
